# EUROPEAN PATENT APPLICATION

(11) **EP 4 535 365 A1**
(43) Date of publication of application: **09.04.2025**
(21) Application number: 23827161.3
(22) Date of filing: 19.06.2023
(51) Int. Cl.: G16H 10/20

(54) **CLINICAL TRIAL ASSISTANCE METHOD AND CLINICAL TRIAL ASSISTANCE SYSTEM**

(30) Priority: 21.06.2022 JP 2022099352
(71) Applicant: Japanese Foundation For Cancer Research, Tokyo 135-8550 (JP)
(72) Inventor: SUZUKI Kazuhiro, Tokyo 135-8550 (JP); OGUCHI Masahiko, Tokyo 135-8550 (JP)
(74) Representative: Prüfer & Partner mbB Patentanwälte · Rechtsanwälte
(86) International application number: PCT/JP2023/022600
(87) International publication number: WO 2023/248978

(57) **Abstract**

Candidates for a clinical trial can be efficiently narrowed down by, after converting eligibility conditions and exclusion conditions of the clinical trial into search words, performing screening using a database in which structured medical examination data is stored. It is possible to provide a method and a system that select candidates for a clinical trial and a therapeutic trial more efficiently with less work.

## Description

### Technical Field

The present invention relates to a method for supporting a clinical trial and particularly relates to design of a clinical trial, a method for screening patients that meet eligibility criteria of participants of a therapeutic trial, and a clinical trial assistance system.

### Background Art

Heretofore, subjects of a clinical trial and a therapeutic trial are recruited mainly from patients who are hospitalized in or visit a medical institution in which a trial is to be performed. Specifically, a physician selects patients who can be candidates from patients for whom the physician makes a diagnosis, or a clinical research coordinator searches for patients who become candidates while confirming content of medical records. Alternatively, there is also a case where patients who become candidates are selected from a patient group registered as a patient cohort. Specifically, patients who become candidates are selected by comparing eligibility conditions and exclusion conditions of a trial protocol complied in approximately several pages to ten pages, with clinical data and determining whether or not patients meet conditions of the clinical trial.

The eligibility criteria of the therapeutic trial are determined in detail, and determination of eligibility requires accuracy and strictness, and thus, it requires an enormous amount of time to confirm whether each of the patients meets the eligibility criteria. Thus, there is a problem that requires a substantial amount of work for a physician or a clinical research coordinator to select candidates for the therapeutic trial from patients who visit or are hospitalized in the medical institution.

Further, a pharmaceutical company is allowed to provide information to subjects of the therapeutic trial, and thus, the subjects are recruited widely through newspapers, magazines, television and the Internet. However, also in this case, a work of checking clinical data of candidates against the eligibility criteria is performed in a similar manner. In either case, the work is extremely troublesome and requires accuracy and strictness.

Selection of patients who are to participate in the clinical trial is mainly performed by human power and remains inefficient in both cases of a case where the work is performed by the medical institution and a case where the work is performed by the pharmaceutical company, and it is said that in a case of therapeutic trials that require collection of many subjects, trials for which the target number of subjects can be collected by a certain due date is equal to or fewer than half. If patients of the number required for the therapeutic trial cannot be collected, an implementation plan is forced to be changed, which may cause large cost burden as well as delay of the therapeutic trial. Thus, determination of the eligibility conditions and the exclusion conditions is an important element in making the implementation plan. While there is a case where the eligibility conditions and the exclusion conditions are determined with reference to conditions of a similar clinical trial, it is extremely difficult to estimate the number of patients who meet various conditions determined in detail. While the pharmaceutical company requires to conduct an interview with each medical institution for which the therapeutic trial is to be requested as to the number of patients who can be candidates of the therapeutic trial in advance before the therapeutic trial is started, it is currently difficult to predict a rough number of patients. Thus, many problems such as delay of the therapeutic trial and increase in cost arise, which, as a result, lead to a main factor of requiring an enormous amount of time to develop medicines.

Thus, a method for screening patients who meet eligibility criteria of a clinical trial and a method for creating eligibility criteria has been proposed in related art (Patent Literatures 1 to 5). Patent Literature 1 discloses a method for screening patients eligible for a therapeutic trial by checking prescriptions and prescription information prescribed for patients against information indicating eligibility criteria of the therapeutic trial. In this method, in a case where information that identifies medical institutions which create the prescriptions is included, it is possible to estimate an approximately number of patients who can be targets in each of the medical institutions. Patent Literature 2 discloses a system including input means for allowing a physician to input data of candidates for a subject, subject selection means for verifying data to select a subject, and information processing means for collecting data of the selected subject. Patent Literature 3 is directed to identify the number of potential researchers from expertise regarding diseases, past results in clinical trials, and the like, and estimating the number of subjects of a clinical trial. Patent Literature 4 discloses a system and a method for creating incorporation (eligibility criteria)/exclusion criteria based on quantitative analysis to create an implementable clinical trial implementation plan. Patent Literature 5 discloses a matching device that searches for a therapeutic trial that matches a patient based on information regarding the patient.

### Citation List

### Patent Literature

Patent Literature 1
   Japanese Patent Laid-Open No. 2016-24663
Patent Literature 2
   Japanese Patent Laid-Open No. 2002-183320
Patent Literature 3
   Japanese Translation of PCT International Application Publication No. 2021-523478
Patent Literature 4
   Japanese Translation of PCT International Application Publication No. 2021-533453
Patent Literature 5
   Japanese Patent Laid-Open No. 2021-111248
Patent Literature 6
   Japanese Patent Laid-Open No. 2019-133540

### Summary of Invention

### Technical Problem

However, in the invention disclosed in Patent Literature 1, prescriptions and prescription information are checked, and thus, merely name of the disease can be inferred, and none of the information regarding treatment methods such as an operation that the patient has been undergone so far can be obtained. Thus, the information is incomplete, and burden on a physician or a coordinator who screens patients is not reduced. The invention disclosed in Patent Literature 2 requires input of data regarding subject candidates to be provided by a physician, and thus does not reduce work of the physician in patient screening. The invention disclosed in Patent Literature 3 is directed to grasping the number of patients who become targets based on the expertise of a physician. While a rough number of candidates for the therapeutic trial can be estimated, selection of patients depends on memory of the physician, and thus, the number of candidates for the therapeutic trial cannot be accurately grasped. Further, with this method, even if the number of candidates for the therapeutic trial can be grasped, work of selecting patients based on the eligibility criteria by the physician cannot be reduced. In the invention disclosed in Patent Literature 4, in order to quantitatively analyze creation of the implementable implementation plan, it is necessary to select and analyze parameters from many parameters including disease/type of failure/stage, age, gender and a phase of a clinical trial. Selection of the parameters is not necessarily unambiguous, and whether the implementation plan can be created while optimal eligibility criteria are selected cannot be verified. The invention disclosed in Patent Literature 5 is a system that searches for a trial that matches a patient and is not a system for reducing work of a pharmaceutical company to secure subjects and work of a physician to determine whether the patient meets eligibility criteria. Further, also in a case where a therapeutic trial that matches a patient is searched for, the system does not cooperate with a database in which patient information is stored, and thus, it is necessary to register patient information, which requires a lot of work.

A problem to be solved by the present invention is to provide a method for setting eligibility conditions and exclusion conditions upon creation of an implementation plan of a clinical trial and determining design of the clinical trial, and a system. Further, a problem to be solved by the present invention is to provide a screening method for selecting clinical trial subjects more efficiently with less work, and a system. As a result of an appropriate clinical trial design being created, it is possible to collect patients of the number necessary for the clinical trial and perform the clinical trial along an implementation plan, which leads to shortening of a recruiting and entry period of clinical trial candidates and reduction in development cost. In a medical institution, a physician or a clinical research coordinator performs work of checking and confirming patient information of the patients screened in advance against the eligibility conditions and the exclusion conditions, so that it is possible to substantially reduce the number of patients to be confirmed and the patient information, and substantially reduce a work of selecting candidates. Further, also in a case where therapeutic trials appropriate for individual patients are searched for, it is only necessary to perform search as to whether or not there is a therapeutic trial that matches the patient information, so that a result can be promptly obtained.

### Solution to Problem

The present invention relates to a method for screening candidates for a clinical trial and a therapeutical trial, and a system, described below.
(1) A clinical trial candidate screening method for screening candidates for a clinical trial, includes searching a database with search words, and extracting eligible persons.
   By converting eligibility conditions and exclusion conditions of the clinical trial into the search words and performing screening, it is possible to promptly and efficiently narrow down candidates for the clinical trial.
(2) A clinical trial candidate screening system that screens candidates for a clinical trial, includes a server computer in which patient information is accumulated, an electronic health record system including a data warehouse, a terminal to which patient information is input from each diagnosis and treatment department, and a clinical trial database, in which the patient information input from the electronic health record system and the terminal at each diagnosis and treatment department is accumulated in the server computer, search words converted from eligibility conditions and exclusion conditions of clinical trial information are accumulated in the clinical trial database, and the accumulated patient information is searched with the search words, thereby a matching patient can be extracted.

The present system is a system capable of promptly extracting candidates for a clinical trial by converting the clinical trial information into search words and searching the patient information. The present system can be used to determine whether the created eligibility conditions and exclusion conditions of the clinical trial are appropriate, analyze adverse events of medicine after approval, perform search as to whether or not there is a clinical trial that matches a patient, and the like, as well as being used to extract candidates for the clinical trial.

### Brief Description of Drawings

[Figure 1] Figure 1 is a view schematically illustrating start of a clinical trial to selection of matching patients.
[Figure 2] Figure 2 is a view indicating a step of selecting candidates for a clinical trial within a medical institution of the present embodiment.
[Figure 3] Figure 3 is a view schematically illustrating a clinical trial candidate screening system of the present embodiment.
[Figure 4] Figure 4 is a view indicating an example of items to which medical examination information is to be input as structured information of the present embodiment, where ● indicates items to be used for screening the candidates for the clinical trial.
[Figure 5] Figure 5 is a flowchart indicating a confirmation process of patient confirmation/consent matters of the present embodiment.
[Figure 6] Figure 6 is a view indicating a step of selecting a clinical trial that matches a patient of the present embodiment.

### Description of Embodiment

The present invention relates to a method for selecting candidates for a clinical trial from patient information stored in a database of a medical institution. The clinical trial is an intervention study to be performed within a framework of clinical research, and a trial to be performed for the purpose of obtaining approval as a medicine, medical equipment or a regenerative medical product from the Ministry of Health, Labor and Welfare among the clinical trials is defined as a therapeutic trial. In other words, the therapeutic trial is concept included in the clinical trial. The present invention is a method and a system for screening target patients promptly with less work in clinical research, particularly in a clinical trial and a therapeutic trial. Particularly, the present invention is an effective system capable of reducing work of a physician and a clinical research coordinator and reducing cost and a period required for a therapeutic trial in the therapeutic trial that requires many candidates. Note that candidates for clinical research are also selected after eligibility criteria and exclusion criteria are determined, and thus, the candidates can be promptly extracted by using the present system.

The present invention exerts a great effect by using a structured medical examination data integrated management database (Patent Literature 6) that structures medical examination information in a medical institution and performs integrated management and support. Structuring of data refers to a data format that is expressed with rows and columns, where each column has unique meaning, and is a data format to be managed based on a unique rule such as a value of the column being selected from specific options, or a numerical value having designated accuracy being input. Further, an aggregate of data items expressed by a combination of a row and a column has unique meaning (for example, patient basic information, pathological information, operation information) and is managed while being distinguished from data having other meaning. As a result of the data being structured for each category and condition and being linked, it becomes possible to grasp a comprehensive state of a patient in a short period of time and search for similar cases, and the like. In a case where data is not structured, it is possible to extract matching patients by converting the data so as to enable search of eligibility conditions and exclusion conditions.

In the present specification, a method and a system targeted at a therapeutic trial of a cancer for which a wide range of eligibility conditions and exclusion conditions are to be set will be described. In medical examinations of a cancer, multidisciplinary treatment combining various treatment methods is often performed in accordance with a type and a stage of the cancer instead of operative therapy, medication therapy, radiation therapy, immune therapy, and the like, being independently performed. Thus, the eligibility conditions and the exclusion conditions of the therapeutic trial are defined in detail with treatment history of the patient so far, gene mutation, and the like, as well as name of a disease (site of the disease) and a state of the disease such as a stage of the disease, and it requires a lot of work for a physician to select a patient that meets eligibility criteria. If the method and the system can be targeted at the therapeutic trial of a cancer, it is obvious that the method and the system can also be applied to therapeutic trials of other diseases. The present invention will be described below using examples.

### [Step of Selecting Candidates for Clinical Trial]

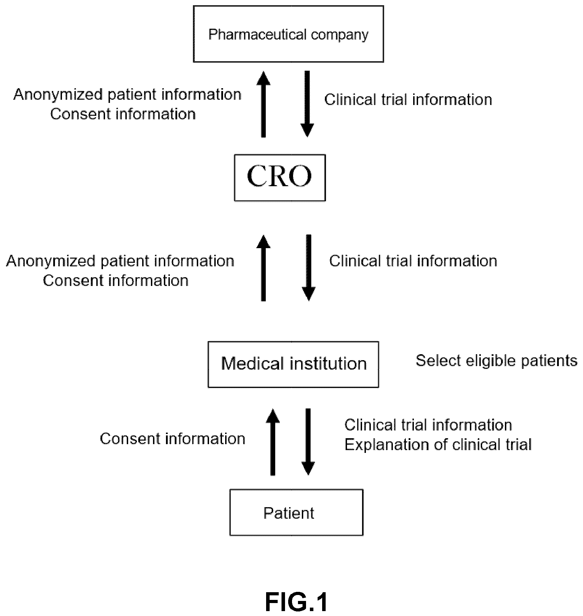

Outline from start of a clinical trial until selection of matching patients will be described first (Figure 1). A pharmaceutical company describes eligibility conditions and exclusion conditions for target patients divided into items when a clinical trial is performed. For example, in a case where "patients with advanced solid cancers" are made targets, the pharmaceutical company creates a document that defines the eligibility conditions and the exclusion conditions with the sentence such as "patients with cancers recurring after local standard therapy or with cancers that are advanced or for which there is no standard treatment method" and outsources the clinical trial to the Contract Research Organization (CRO). The CRO selects a medical institution having patients that match the clinical trial to be performed and requests implementation of the clinical trial.

The medical institution checks the eligibility conditions and the exclusion conditions for each patient based on clinical trial information from the pharmaceutical company/the CRO and selects matching patients. After a physician, or the like, makes a sufficient explanation regarding the clinical trial and standard treatment to a patient who matches the clinical trial, in a case where the patient selects the clinical trial, a written consent form is obtained, and the clinical trial is started. All the patient information and the consent information are anonymized and reported from the medical institution to the CRO/the pharmaceutical company. Note that the consent form signed by both the physician and the patient is stored within the medical institution.

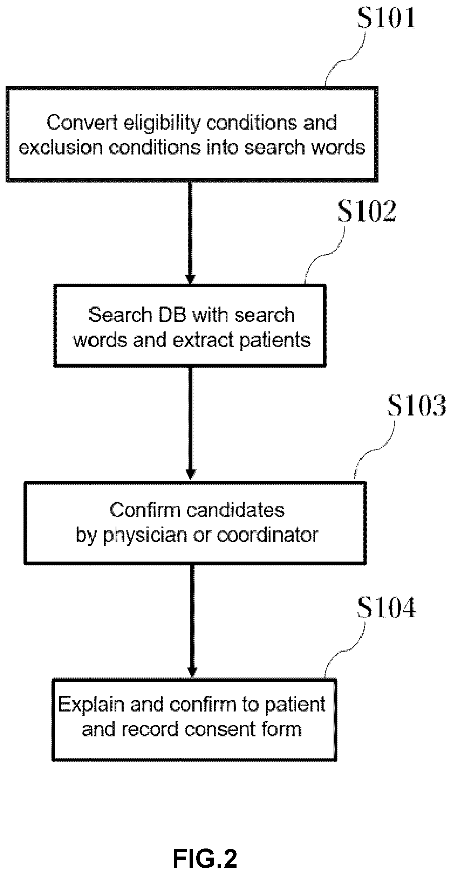

Outline of the step of screening candidates for the clinical trial of the present embodiment will be indicated in Figure 2. It is assumed in the present embodiment that patients are screened using a structured medical examination data integrated management database. Note that the medical examination data structured as described above will be referred to as "structured medical examination data", and a database that performs integrated management of data such as examination, medical examination and hospital records at each department within the medical institution will be referred to as an integrated management database. Further, here, simple expression of an "integrated management database" is synonymous with the structured medical examination data integrated management database because patient information is stored as the structured medical examination data.

Candidates for the clinical trial are selected by checking the eligibility conditions and the exclusion conditions divided into items described in the sentence. The database cannot be searched using the eligibility conditions, and the like, described in the sentence as they are, and thus, the eligibility conditions, and the like, are converted into searchable words, that is, words accumulated in the structured database as structured data (S101). For example, in a case of a therapeutic trial "targeted at patients with advanced solid cancers", the "patients with advanced solid cancers" are unlikely to be described in the medical records, and thus, it is only necessary to set cancers such as a "lung cancer" and a "breast cancer" other than a blood cancer, as search words. Further, regarding "advanced" cancers, a search word that "excludes stages of the disease T1/T2, N0" which are early cancers is set. Also regarding "incurable", it is possible to perform search using whether or not the cancer recurs or metastasizes, treatment history using a medicine that is used in standard treatment, and the like. Further, it is necessary that a definite diagnosis is made when the clinical trial is performed, and thus, whether or not a pathological diagnosis is registered becomes an important selection criterion.

Also in a case where the patient information is not accumulated as the structured data, in a case where the clinical trial is targeted at "patients with advanced solid cancers", it is only necessary to set as search words, types of cancers such as a "lung cancer" and a "breast cancer" and set stage classification such as TNM classification for a stage of the disease. Regarding the condition such as "incurable", while there is a possibility that inconsistency of notation by physicians may become a problem, it is possible to narrow down patients who become targets by performing search while inputting name of medicines, and the like, used in the treatment. Also in a case where the patient data is not accumulated as the structured data, it is only necessary to use a word to be used in a case where the patient data is accumulated as the structured data as the search word.

The eligibility conditions and the exclusion conditions may be converted into search words by one of the pharmaceutical company, the CRO and the medical institution. However, it is necessary to convert the conditions into search words by a person such as a health personnel who can appropriately convert the conditions into medical expression. Alternatively, it is also possible to prepare in advance search words that become candidates as a glossary through natural language analysis by AI, automatically select a search word among the prepared search words and present the search word.

Further, to select the targets, patients who are considered to be alive at the time of search, that is, patients who have visited the medical institution within one year are important because of necessity to select candidates for the clinical trial, and patients aged 18 and older are important because of necessity to obtain consent from a person himself/herself, while these conditions are not directly related to the disease. Outcome, date of last visit, and birth date are recorded as patient basic information, or the like, in the structured medical examination data integrated management database, and thus, also regarding these kinds of information, target date of visit and target birth date are also converted into search words, and patients are screened (S102).

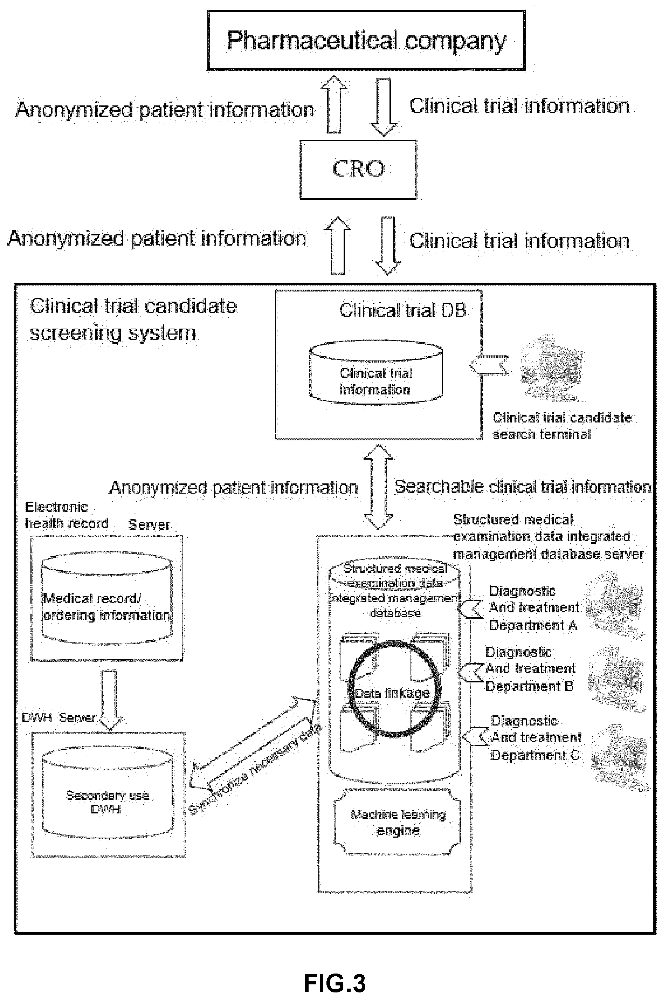

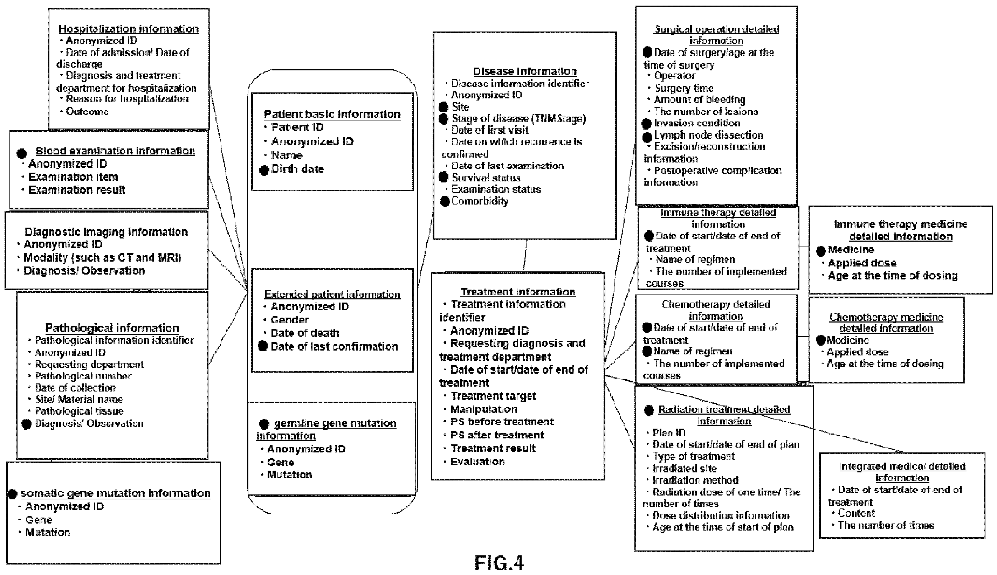

Search for patients using the structured medical examination data integrated management database will be described (Figure 3). The clinical trial information for selecting patients who match the clinical trial, specifically, the eligibility conditions and the exclusion conditions are transmitted from the pharmaceutical company/the CRO to the clinical trial database. The eligibility conditions and the exclusion conditions are converted into search words with which the structured medical examination data integrated management database can be searched, that is, words accumulated as the structured data, and patients that match the conditions are selected. The structured medical examination data integrated management database is a database that stores structured information of the medical institution, specifically, information of structured information input to the electronic health records and the structured information input at each diagnosis and treatment department which are linked. In the structured medical examination data integrated management database, the patient information is managed with anonymized IDs. The server computer includes anonymized ID assignment means for automatically assigning an anonymized ID if a patient ID is registered at a reception, or the like, and personal information of the patient is recorded as patient basic information along with the patient ID, and other information is recorded as extended patient information to which the anonymized ID is assigned in a server storage unit (see Figure 4). Thus, information of the selected patients is all anonymized. The anonymized IDs play an important role to protect personal information of the patients upon the clinical trial or the therapeutic trial. Information input to the electronic health records is stored in a data warehouse (DWH) and synchronized.

Examples of information to be synchronized from the electronic health records can include the following (see Figure 4). The hospitalization information includes information on an anonymized ID, date of admission/date of discharge, a diagnosis and treatment department for hospitalization, a reason for hospitalization, outcome. Further, blood examination information includes an anonymized ID, an examination item and an examination result. Diagnostic imaging information includes an anonymized ID, modality such as CT and MRI, and a diagnosis/observation. Pathological information includes information on an anonymized ID, a requesting department, a pathological number, date of collection, a site/material name, a pathological tissue, and a diagnosis/observation. Somatic gene mutation information includes information on an anonymized ID, a gene and mutation.

Data at each diagnosis and treatment department includes all the information regarding diseases and treatment. Disease information includes information on an anonymized ID, a site, a stage of the cancer (TNM stage), date of the first visit, date on which recurrence is confirmed, date of the last examination, survival status, an examination status, comorbidity. Treatment information includes information on an anonymized ID, a requesting diagnosis and treatment department, date of start date/end date of treatment, a treatment target, manipulation, PS before treatment, PS after treatment, a treatment result, evaluation. More specific treatment information is recorded as information regarding respective kinds of treatment of a surgical operation, immune therapy, chemical therapy, and radiation treatment. Note that when disease information is newly registered, the disease information is managed with an automatically issued identifier (disease information identifier) for uniquely specifying each piece of data.

Further, in a similar manner to anonymization, in order to prevent a specific person from being identified unless the personal information is checked against other information to protect personal information, also in a case where anonym processing information processing of obtaining personal information through processing is performed, it is possible to perform management through similar procedure such as deletion of description included in the personal information or personal identification code, and use the information to grasp the number of patients and select patients.

The treatment information is different in accordance with what kind of treatment is undergone, and thus, segmentalized detailed information is stored in the integrated management database. The detailed treatment information includes surgical operation detailed information, immune therapy detailed information, chemotherapy detailed information, radiation treatment detailed information, and integrated medical detailed information. The surgical operation detailed information includes information on date of surgery/age at the time of surgery, an operator, a surgery time, an amount of bleeding, the number of lesions, an invasion condition, lymph node dissection, excision/reconstruction, postoperative complications, and is linked to the treatment information in the database. Further, the immune therapy detailed information and the chemotherapy detailed information include information on date of start/date of end of treatment, name of regimen, the number of implemented courses, and further include information on a medicine, an applied dose, and age at the time of dosing and is linked to the treatment information in the database. Further, the radiation treatment detailed information includes information on a plan ID, date of start/date of end of the plan, a type of treatment, an irradiated site, an irradiation method, a radiation dose of one time/the number of times, a dose distribution, and age at the time of start of the plan and is linked to the treatment information in the database. Further, how the cancer was found, and a comorbidity can be also selected and input. These kinds of information are structured and stored, so that it is possible to perform search with higher accuracy at a higher speed compared to a typical electronic health record system.

In contrast, in the typical electronic health record system, while information on medical examination and treatment such as symptoms of a patient who visits the medical institution, observation, an examination result, a diagnosis, treatment and prescriptions is sequentially input and recorded in an electronic health record along with an ID of the patient and personal information by a health personnel such as a physician in the medical institution, there are a wide variety of data items to be managed and formats thereof in the respective departments. In the medical institution, electronic health records of all patients are normally stored and protected as they are. Further, description of disease sites and symptoms is often different for each health personnel, which inhibits search. Still further, the information is made a database individually in the respective diagnosis and treatment departments, and forms of stored data are often not made uniform. Records of daily medical examination by the typical electronic health record system are data stored in the database as is in natural language and are not structured, which inhibits selection of candidates for the therapeutic trial. In the structured medical examination data integrated management database, the patient information is structured, and the anonymized ID is assigned, which are extremely advantageous to selection of patients who match the clinical trial.

An example of search using the structured medical examination data integrated management database will be described next. ● in Figure 4 indicates items available for search for selecting candidates for the therapeutic trial. For example, in a case of the therapeutic trial targeted at "patients with advanced solid cancers", TNM classifications and sites are respectively stored in the stage of the disease (TNM stage) and the site of the "disease information" as structured information, and thus, patients with advanced cancers can be easily selected. Further, concerning whether a definitive diagnosis that is important in the therapeutic trial is made, it is possible to determine whether or not pathological diagnosis is registered from whether or not there is description of the diagnosis/observation of the "pathological information". Still further, it is possible to obtain patients who are considered to be alive at the time of search, for example, from whether or not the patients are alive in the "disease information" of patients who have visited the medical institution within one year, and obtain patients aged 18 and older who can be candidates by consent from persons themselves from information on the birth dates of the "patient basic information".

Whether the patients screened with the eligibility conditions and the exclusion conditions are eligible as candidates for the therapeutic trial are determined by a physician or a clinical research coordinator (Figure 2, S103). The patients are screened, and thus, an attending physician or the clinical research coordinator only requires confirming whether individual patients are eligible as candidates for the therapeutic trial, so that the candidates can be selected with less work.

Note that in the structured medical examination data integrated management database, the patient information is anonymized as described above, and search can be performed using the anonymized IDs, so that it is possible to select patients while preserving anonymity. The pharmaceutical company requires to set appropriate eligibility conditions and exclusion conditions for collecting patients of the number required for the therapeutic trial upon creation of an implementation plan of the therapeutic trial. The pharmaceutical company can determine in advance an approximate number of candidates for the therapeutic trail in the medical institution with the set eligibility conditions and exclusion conditions, and thus, can set appropriate conditions and make use of the number in creation of the therapeutic trial plan.

Further, in the structured medical examination data integrated management database, in a case where a staff member within the medical institution accesses the patient information, the patient ID and name can be displayed. It is therefore possible to smoothly explain the therapeutic trial to the patient, acquire a consent form, or the like. Further, access from outside the medical institution can be determined from an IP address or a route of communication packets, and thus, setting can be made so that only the anonymized ID is displayed to access from outside the medical institution, or even within the medical institution, to staff members of the CRO, and the like, who do not have access authority to the structured medical examination data integrated management database.

Further, the present system includes an interface and a repository conforming to HL7 Fast Health Interoperable Resources (FHIR) that is medical information standards and is constructed to be able to coordinate with systems outside the medical institution such as the CRO and the pharmaceutical company and various kinds of systems within the medical institution online and offline. It is therefore possible to smoothly exchange the therapeutic trial information.

In a case of a typical electronic health record system in which the medical examination information is not stored as structured data, it is necessary to set search words in consideration of inconsistencies of notation, and the like, in description of electronic health records by respective physicians. Even in a database that is not structured, it is possible to roughly screen patients by appropriately selecting search words such as name of diseases, and name of medicines that are being used for treatment. Further, for processing of structuring information described in the electronic health records, it is also possible to perform natural language processing using artificial intelligence during data processing work and store information necessary for searching eligibility conditions of the therapeutic trial as structured data in advance.

Further, in a case of a typical electronic health record system, there is a case where information from a department different from a department of a physician who is in charge of the treatment, such as pathological information and examination information, is stored in a database different from the electronic health record system. In such a case, a step of extracting information that is desired to be structured, for example, name of a site of the pathological report, name of the pathological diagnosis, observation, and the like, from each database, importing the information into a work database and performing extraction, and the like, is required.

Then, a physician needs to perform explanation to a patient selected as a candidate for the therapeutic trial, obtain a consent for the therapeutic trial, obtain a consent for biopsy to be performed during the therapeutic trial, provision of a specimen obtained by the biopsy, and the like, and keep records (Figure 2, S104). The patient can be registered as a subject of the therapeutic only after these consents can be obtained.

### [Reliability Guarantee Check System]

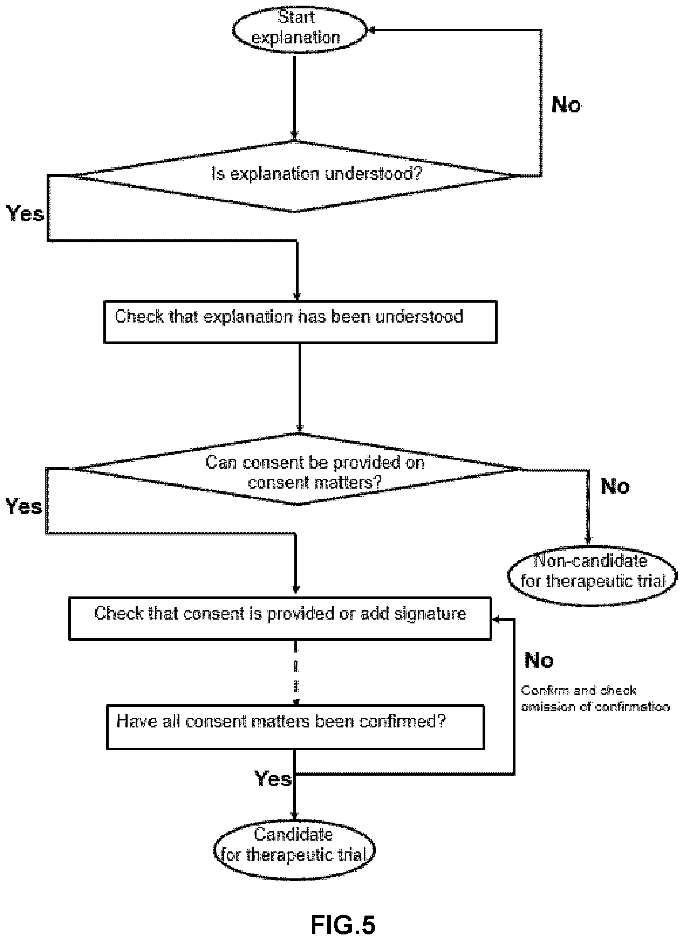

In application of approval of the therapeutic trial, and the like, reliability guarantee operation of examining whether data attached to an application form is collected and created in accordance with the "standards specified by the Minister of Health, Labor and Welfare" and the "standards for reliability of application data" is performed. The consent form of the subject of the therapeutic trial secures reliability of the therapeutic trial and thus is an extremely important document. If confirmation to the subject or obtaining of a consent form is omitted, the reliability of the therapeutic trial is impaired. Thus, while there are many matters to be confirmed to the subject and many matters for which consents should be obtained, it is necessary to certainly perform these and organize and store records. A function of recording respective work procedures as a log according to a standard work procedure manual is necessary in terms of reliability guarantee, security management and securing transparency of the procedure. The clinical trial candidate screening system includes a checking mechanism for preventing oversight in confirmation (Figure 5).

Confirmation to the patient is started from explanation by the physician or the clinical research coordinator. In a case where the patient can understand the explanation, the procedure proceeds, and in a case where the patient has questions, the questions are cleared up by the explanation being performed again. In a case where the patient understands the explanation, a checkbox is marked, and the matters for which consents should be obtained are confirmed one by one, consent fields are checked or a signature is added, and in a case where consents can be obtained for all the matters, the procedure proceeds to registration as a candidate for the therapeutic trial. The patient cannot be a candidate for the therapeutic trial if there is any matter that the patient cannot provide consent. The therapeutic trial may be explained face-to-face by an explainer or an interview may be conducted through the web, or the like. Further, the patient may receive quick explanation by an avatar of a physician, may express his/her intention to provide consent for a portion for which the patient can provide consent, and may receive explanation from a health personnel for only a portion for which the patient desires to receive further explanation.

By performing confirmation on the consent matters using an electronic consent form on which an electronic signature can be added, it is possible to prevent oversight of checking in the consent fields and omission of explanation. Further, if explanation can be performed and consent can be obtained through the web, even in a case where a candidate for the therapeutic trial is located in a remote place, the candidate can be registered for the therapeutic trial. In a case where the electronic consent form is used, a patient operates a terminal and checks the consent fields while confirming the matters one by one. In a case where there is a matter for which confirmation cannot be received, oversight of checking is displayed on an operation terminal, and thus, there is no concern that matters that should be confirmed are overlooked. In a case where confirmation can be received for all the matters, the patient is registered as the candidate for the therapeutic trial. By checking the matters that should be checked in accordance with a form created by the pharmaceutical company in advance, it is possible to perform checking without omission and certainly collect consent forms. Records indicating that explanation has been performed to the patient, and the patient has confirmed the matters and has provided consent are recorded in the electronic health record system and can be imported to the structured medical examination data integrated management database as necessary.

Further, patient monitoring that is the most important work during the therapeutic trial can be also performed via the web, and the like. For example, it is also possible to efficiently collect as a monitoring dataset during the therapeutic trial, information and data when the subject of the therapeutic trial participating from a remote place receives monitoring after administration of the medicine by connecting to the present system.

### [Creation of Therapeutic Trial Report]

In the present system, a therapeutic trial report can be easily created. A new therapeutic medicine is often developed through an international joint therapeutic trial, in which case, forms from registration of a patient to a report of a case require to be set according to international standards. In recent years, a format based on international clinical research data exchange standards, called Clinical Data Interchange Standards Consortium (CDISC) is recommended, and the regulatory authorities admit acceptance of CDISC data. Thus, the electronic health records and reports of cases in the medical institution desirably have specifications that enable transition and exchange to the CDISC format. If a report converted into English can be output from the integrated management database in accordance with the format based on the CDISC after the clinical trial or the therapeutic trial ends, it is possible to seamlessly deal with the international joint therapeutic trial.

### [Extraction of Synthetic Control Arm Group]

In the clinical trial, a group comparison test like a randomized controlled trial, in which subjects are assigned to a treatment group and a placebo group or a standard treatment group, is performed. While accurate data can be obtained through the group comparison test, in a case where the trial is performed while the subjects are divided into two groups, there is a problem that a large number of subjects are required, and it takes time and cost. Further, in a case of a life-threatening disease, in a case where a treatment effect in one group is excellent, an opportunity of the patient to make a decision for treatment is lost, and thus, an ethical problem arises. To avoid such problems, a single-arm trial in which a trial is performed only in a single group without providing a comparison group is performed. While the single-arm trial, in which a trial is performed while a certain threshold is provided to an expected effect, is superior to the group comparison test in terms of time and cost, there is no comparison, and thus, scientific reliability is questioned. Synthetic Control Arm (SCA) is a therapeutic trial design that has been recently advocated as an option of the therapeutic trial design which does not require subjects to be compared, or which can reduce the number of targets.

In the therapeutic trial, a comparison group of a therapeutic medicine/treatment method for which the trial is to be performed, is standard treatment. In the SCA, patients who have received standard treatment in the past for the same indication are extracted as targets. For example, in a similar manner to the above-described example, in a case where a comparison group is screened in the therapeutic trial targeted at "patients with advanced solid cancers", patients for whom outcome has been known regardless of whether the patients are alive or dead at the time of search are narrowed down by using survival status and date of last confirmation of the "extended patient information". It is only necessary to determine whether or not the pathological diagnosis is registered from whether or not there is description of diagnosis/observation in the "pathological information" as to whether or not a definitive diagnosis is made, screen the patients with the stage of the disease (TNM Stage) and the site in the "disease information" to obtain patients with advanced solid cancers, and screen a group in which treatment is performed with the standard treatment that should be made a target in the surgical operation detailed information, the immune therapy detailed information, the chemical therapy detailed information, the radiation treatment detailed information, and the like, for the treatment method.

By using the SCA, the standard treatment is not assigned to patients who want to participate in the therapeutic trial of a new medicine, and thus, an opportunity to make a decision for the treatment is not lost. Further, the pharmaceutical company can obtain a result similar to that obtained through the group comparison test while reducing cost and time required for the therapeutic trial. According to the system of the present invention, it is possible to promptly screen patients who become targets of the SCA.

### [Selection of Therapeutic Trial that Matches Patient]

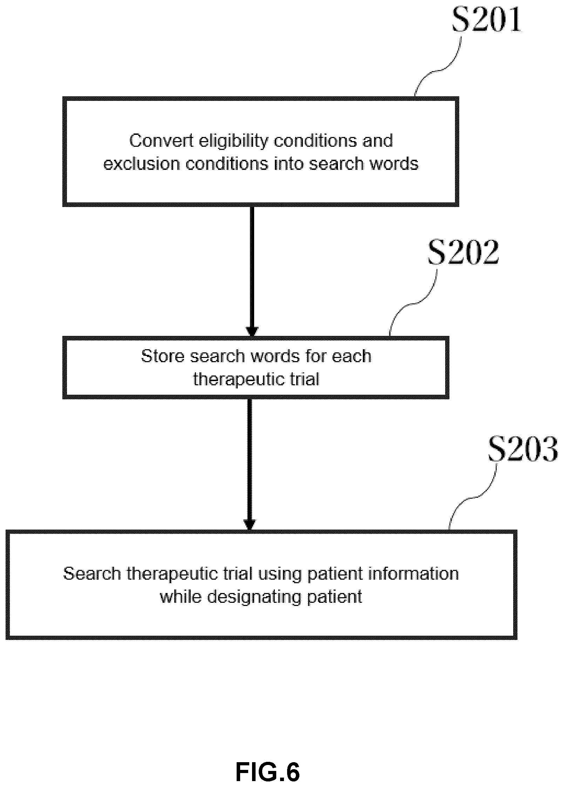

The present system can search for a therapeutic trial that can be entered by a specific patient as well as selecting patients who match a therapeutic trial (Figure 6). Eligibility conditions and exclusion conditions for selecting patients that match a clinical trial are converted into searchable words in a stage in which implementation of the clinical trial is considered (Figure 6, S201), and the clinical trial information converted into the searchable words is stored in a clinical trial database of a clinical trial candidate screening system for each clinical trial (S202). The patient information is structured and stored in the structured medical examination data integrated management database, and thus, in a case where a specific patient searches for a clinical test that can be entered by the patient, the patient only requires to confirm matching between the patient data and the search words for each clinical trial stored in the clinical trial database (S203). The system that searches for a clinical trial that matches each patient expands a possibility of selecting a new treatment method.

### [Study of Adverse Event]

During the therapeutic trial, examination values such as a blood examination value and a urine examination value, and results of interviews by a physician and a pharmacist are recorded for patients for whom a therapeutic medicine has been administered, and adverse events are collected and reported. After the medicine is approved, early postmarketing phase vigilance is performed in the first six months after start of sale, adverse events are recorded in detail, and measures for safety are taken. However, if the medicine is used by many patients after the medicine is manufactured and sold, it is difficult to obtain detailed data as during the therapeutic trial. The present system can also survey adverse events of patients after the medicine is approved. It is possible to perform analysis whether specific tendency can be found by screening the patient group in which the target medicine has been administered from the treatment information and extracting examination values such of blood examination, urine examination, and the like. Further, events not recorded as examination values, for example, adverse events such as diarrhea, hand-foot syndrome, and numbness can be extracted by being structured and recorded. If the adverse events can be extracted, a patient group with a high risk for a specific adverse event can be specified, so that the medicine can be more safely used.

As described above, the present system is a system capable of promptly determining eligibility conditions and exclusion conditions necessary for creating an implementation plan of a clinical trial, screening patients who match the clinical trial, considering adverse events, and searching for a clinical trial appropriate for a patient. Further, the embodiment described above is merely an example, and it goes without saying that various changes, combinations of embodiments, omission, and the like, can be made within a range not deviating from the gist of the invention.

## Claims

1. A clinical trial candidate screening method for screening candidates for a clinical trial, the clinical trial candidate screening method comprising:
searching a database with search words; and
extracting eligible persons.

2. The clinical trial candidate screening method according to claim 1, wherein data is stored in the database as structured data.

3. The clinical trial candidate screening method according to claim 2, wherein
clinical trial information is explained to the selected candidates, and
consent matters are electronically recorded.

4. A synthetic control arm (SCA) target screening method for screening patients who have undergone standard treatment for a new medicine for which a clinical trial is to be performed as targets of SCA, the SCA target screening method comprising:
searching a database with search words;
extracting patients who have undergone the standard treatment in the past as targets; and
selecting eligible persons from the targets.

5. The SCA target screening method according to claim 4,
wherein data is stored in the database as structured data.

6. A clinical trial candidate screening system that screens candidates for a clinical trial, the clinical trial candidate screening system comprising:
a server computer in which patient information is accumulated;
an electronic health record system including a data warehouse;
a terminal to which the patient information is input from each diagnosis and treatment department; and
a clinical trial database, wherein
the patient information input from the electronic health record system and the terminal at each diagnosis and treatment department is accumulated in the server computer,
search words converted from eligibility conditions and exclusion conditions of clinical trial information are accumulated in the clinical trial database,
the accumulated patient information is searched with the search words, and
a matching patient can be extracted.

7. The clinical trial candidate screening system according to claim 6, wherein the patient information is accumulated in the server computer as structured patient information.

8. The clinical trial candidate screening system according to claim 6 or 7, wherein personal information of a patient is stored in a server storage unit along with a patient ID and an anonymized ID.

9. The clinical trial candidate screening system according to claim 6 or 7, comprising a mechanism that creates and checks an electronic consent form, the mechanism confirming and recording consent matters for the candidates for the clinical trial.

10. The clinical trial candidate screening system according to claim 6 or 7, comprising a mechanism that performs anonym processing information processing of the patient information so that subjects can be selected while protecting personal information.

11. A method, comprising:
converting eligibility conditions and exclusion conditions of a clinical trial into search words;
extracting patients who match the conditions using the clinical trial candidate screening system according to claim **6;** and
determining whether the eligibility conditions and the exclusion conditions of the clinical trial are appropriate by grasping the number of patients who match the conditions.

12. An adverse event analysis method for analyzing adverse events by a medicine, the adverse event analysis method comprising:
extracting patients who have used a medicine for which analysis is to be performed using the clinical trial candidate screening system according to claim 6; and
analyzing examination values, observation and symptoms at the time of use of the medicine.

13. A search method for searching a clinical trial that matches a patient, the search method comprising:
referring to data that is structured data of patient information of the patient using search words converted so as to enable search, from clinical trial information accumulated in the clinical trial database according to claim 6 for each clinical trial;
extracting a matching clinical trial.
